# EUROPEAN PATENT APPLICATION

(11) **EP 4 075 370 A1**
(43) Date of publication of application: **19.10.2022**
(21) Application number: 21750284.8
(22) Date of filing: 04.02.2021
(51) Int. Cl.: G06Q 50/02

(54) **LIVESTOCK INFORMATION MANAGEMENT SYSTEM, LIVESTOCK INFORMATION MANAGEMENT SERVER, LIVESTOCK INFORMATION MANAGEMENT METHOD, LIVESTOCK INFORMATION MANAGEMENT PROGRAM, AND DATA STRUCTURE FOR LIVESTOCK INFORMATION MANAGEMENT**

(30) Priority: 07.02.2020 JP 2020019349
(71) Applicant: Eco-Pork Co., Ltd., Setagaya-ku Tokyo 154-0016 (JP)
(72) Inventor: ARAFUKA Shinsuke, Tokyo 154-0016 (JP)
(74) Representative: Fédit-Loriot
(86) International application number: PCT/JP2021/004151
(87) International publication number: WO 2021/157663

(57) **Abstract**

A livestock information management system 1 for managing breeding of multiple mother pigs includes: an information storage unit 121 configured to store mother pig identifiers D11 for identifying the respective mother pigs and mother pig status information D12 including mother pig statuses indicating rearing states of the mother pigs whose statuses transition by conducting a breeding event on the mother pigs identified by the mother pig identifiers and a history indicating timings at which the mother pig statuses have transitioned, in association with each other; a mother pig extraction unit 111 configured to extract multiple mother pigs in the same mother pig status by using the mother pig status information; and a production group registration unit 113 configured to assign a production group identifier D21 for identifying the mother pigs as a production group on which the same breeding event is to be conducted at the same timing in association with the mother pig identifiers extracted by the mother pig extraction unit.

## Description

### TECHNICAL FIELD

The present disclosure relates to a livestock information management system, a livestock information management server, a livestock information management method, a livestock information management program, and a data structure for livestock information management, which are for managing records of information on works (events) such as mating (breeding), a pregnancy test, childbirth, or weaning conducted on livestock in a livestock industry.

### BACKGROUND ART

In a livestock industry, in order to ensure food safety, manage health of livestock, and efficiently increase productivity, computerization of livestock information management has been performed. Typically, reared livestock has been managed, but a user needs to conduct works such as recording of information on paper in a livestock barn or the like and inputting of information into a computer. However, several thousands to several tens of thousands of livestock are handled depending on the type of livestock. Thus, management of an enormous number of pieces of history information causes problems such as an increase in management load and the possibility of erroneous input of information.

Patent Document 1 describes the following technique as a technique for reducing loads of works of inputting livestock industry history information at livestock farming places. When the user specifies a date and a group and performs the operation of inputting feeding and medication histories, a plan corresponding to the specified group is referred to, feeding and medication schedules close to the specified date are recognized, and scheduled feed and drug names are presented as input candidates. Then, when the user checks and confirms them, the history of use of the scheduled feed and drug names presented as the input candidates are automatically registered in a history data storage unit.

### CITATION LIST

### PATENT DOCUMENT

PATENT DOCUMENT 1: Japanese Unexamined Patent Publication No. 2006-158280

### SUMMARY OF THE INVENTION

### TECHNICAL PROBLEM

However, the technology described in Patent Document 1 is to manage feeding and the like by grouping non-breeding livestock to be fattened, i.e., porkers, and is not capable of group-managing breeding using breeding livestock, in particular, mother pigs.

The present disclosure was made to solve such a problem, and is intended to provide a livestock information management system, a livestock information management server, a livestock information data structure, a livestock information management method, and a livestock information management program, which can manage rearing states of many mother pigs and by which a user can recognize and manage important information in livestock industry business.

### SOLUTION TO THE PROBLEM

In order to achieve the objective, a livestock information management system for managing breeding of multiple mother pigs includes: an information storage unit configured to store mother pig identifiers for identifying the respective mother pigs and mother pig status information including mother pig statuses indicating rearing states of the mother pigs whose statuses transition by conducting a breeding event on the mother pigs identified by the mother pig identifiers and history information indicating timings at which the mother pig statuses have transitioned, in association with each other; a mother pig extraction unit configured to extract multiple mother pigs in the same mother pig status by using the mother pig status information; and a production group registration unit configured to assign a production group identifier in association with the mother pig identifiers extracted by the mother pig extraction unit, the production group identifier identifying the mother pigs as a production group on which the same breeding event is to be conducted at the same timing.

In order to achieve the objective, a livestock information management server for managing breeding of multiple mother pigs includes: an information storage unit configured to store mother pig identifiers for identifying the respective mother pigs and mother pig status information including mother pig statuses indicating rearing states of the mother pigs whose statuses transition by conducting a breeding event on the mother pigs identified by the mother pig identifiers and a history indicating timings at which the mother pig statuses have transitioned, in association with each other; a mother pig extraction unit configured to extract multiple mother pigs in the same mother pig status by using the mother pig status information; and a production group registration unit configured to assign a production group identifier in association with the mother pig identifiers extracted by the mother pig extraction unit, the production group identifier identifying the mother pigs as a production group on which the same breeding event is to be conducted at the same timing.

In order to achieve the objective, a livestock information management method for managing breeding of multiple mother pigs by a computer includes: a mother pig extraction step of extracting, by a mother pig extraction unit, multiple mother pigs in the same mother pig status by using mother pig identifiers for identifying the respective mother pigs, mother pig statuses indicating rearing states of the mother pigs whose statuses transition by conducting a breeding event on the mother pigs, and a history indicating timings at which the mother pig statuses have transitioned; and a production group registration step of assigning, by a production group registration unit, a production group identifier for identifying the mother pigs as a production group on which the same breeding event is to be conducted at the same timing in association with the mother pig identifiers extracted by the mother pig extraction unit.

In order to achieve the objective, a livestock information management program for managing breeding of multiple mother pigs causes a computer to execute: a mother pig extraction step of extracting, by a mother pig extraction unit, multiple mother pigs in the same mother pig status by using mother pig identifiers for identifying the respective mother pigs, mother pig statuses indicating rearing states of the mother pigs whose statuses transition by conducting a breeding event on the mother pigs, and history information indicating timings at which the mother pig statuses have transitioned; and a production group registration step of assigning, by a production group registration unit, a production group identifier for identifying the mother pigs as a production group on which the same breeding event is to be conducted at the same timing in association with the mother pig identifiers extracted by the mother pig extraction unit.

### ADVANTAGES OF THE INVENTION

According to the present disclosure including the foregoing means, rearing of mother pig herds which are important in a livestock industry business can be managed while sequentially grasping the statuses thereof.

### BRIEF DESCRIPTION OF THE DRAWINGS

FIG. 1 is a configuration diagram of a livestock information management system.
FIG. 2 is an example of mother pig information stored in an information storage unit.
FIG. 3 is a schematic diagram illustrating a production group.
FIG. 4 is a flowchart illustrating the flow of processes of a first embodiment.
FIG. 5 is an example of a screen for registering a production group.
FIG. 6 is an example of a screen for registering a newborn baby group.
FIG. 7 is a flowchart illustrating the flow of processes of a second embodiment.
FIG. 8 is a flowchart illustrating the flow of processes of a third embodiment.
FIG. 9 is an example of a screen indicating dynamics of the production group.
FIG. 10 is an example of a screen indicating dynamics of the production group including prediction.
FIG. 11 is a flowchart illustrating the flow of the processes of the third embodiment.
FIG. 12 is a schematic block diagram illustrating a configuration of a computer.

### DESCRIPTION OF EMBODIMENTS

Embodiments will now be described below with reference to the drawings.

In the embodiments described below, pigs are mainly taken as an example of management target livestock. In a livestock industry, the number of pigs raised in a single farm may range from several hundreds to several thousands, the burden of managing such an enormous number of livestock is large, and events and the like may be erroneously conducted on the pigs. In particular, mother pigs give birth to piglets to be shipped and exchanged for money, and thus, are an important business resource for pig farmers. Large-scale farmers breed numerous livestock, and conduct of incorrect events has a direct negative impact on management.

First, the usefulness of production group management to be conducted using the present disclosure in a pig breeding business will be described. For comparison, a case where mother pigs are individually managed one by one as in the prior art will be described as an example. Each mother pig is managed on the premise that a physical mother pig identifier such as an ear tag, an IC tag, or a tattoo corresponds to an electronic mother pig identifier stored in a database. When mother pigs are reared such that they come into estrus naturally according to animal physiology and are bred accordingly, childbirths of the mother pigs are concentrated, or timings when a fattening pig herd which includes newborn piglets born of the mother pigs reaches an appropriate weight for shipping are concentrated. Thus, it is not possible to equalize the timings when products of pig farming are obtained. Facilities and workers as resources of farms are limited, and if breeding of pigs is not performed so as to equalize and optimize childbirths of mother pigs while the facilities and the workers are constraint conditions, necessary works may be concentrated in a short period of time, or piglets may be born greatly exceeding the capacity of a weaning pig house, which may lead to destabilization of a livestock industry business. On the other hand, livestock products are living things. Thus, they cannot be managed like parts or stocks produced in factories, and animal physiology must be respected in rearing management from the viewpoint of recent animal welfare. As a matter of course, a rearing event schedule cannot be managed only by farmer's convenience, and it is necessary to manage the livestock industry while considering the physical conditions and states of the livestock. Therefore, as collective management of the mother pigs, it is efficient to collectively manage only mother pigs in the same rearing state (status) as a group of mother pigs on which a breeding event is to be conducted at the same time. It is preferable that in a livestock information management system, how many mother pigs belong to which production group and what state the mother pigs are in are visualized so that multiple production groups can mutually adjust the time when the breeding event is conducted and the user can obtain these pieces of information and make a decision at any timing desired by the user. In the case of pigs in particular, according to Japanese livestock statistics, the number of pigs reared per farm is more than one digit higher than that of cattle, and a breeding cycle is shorter due to a shorter pregnancy period and more births, resulting in a higher number of newborn babies. For example, cattle have a pregnancy period of 280 days to 285 days and give birth basically to one cattle at a time, so that one breeding cycle is close to a year, and a long-term individual management for livestock is suitable. On the other hand, pigs have a pregnancy period of 114 days to 116 days and give birth to more than 10 piglets at a time, and one breeding cycle is a short cycle of about 150 days from breeding to next breeding. Moreover, when there are many mother pigs in different breeding cycles at the same time, short-term collective management is very complicated. Therefore, it is not efficient to individually manage mother pigs like cattle. The livestock information management system 1 described in the present disclosure reduces work loads of farmers, stabilizes a shipping timing, and improves predictability, thereby allowing assistance of a livestock industry business so as to increase and stably obtain results while effectively utilizing resources as an entire farm.

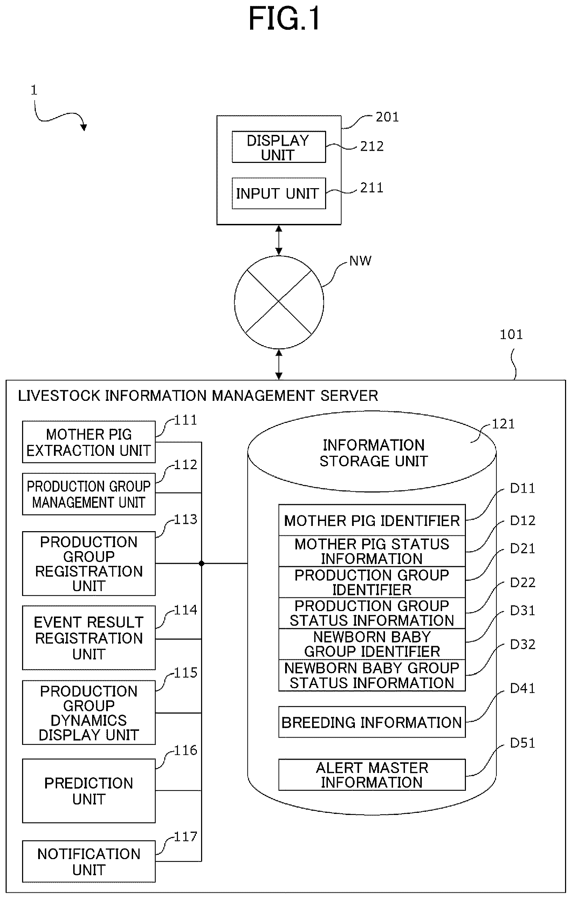

FIG. 1 is a system configuration diagram illustrating a livestock information management system 1 including a livestock information management server 101 according to an embodiment of the present disclosure. As shown in FIG. 1, in the livestock information management system 1 according to the embodiment of the present disclosure, the livestock information management server 101 and a terminal device 201 used by a user are connected to each other so as to communicate with each other via a network NW. The network NW is, for example, a network such as the Internet or the Virtual Private Network (VPN). For simplification of description, FIG. 1 illustrates only one terminal device 201 assuming one user, but the livestock information management server 101 is connectable to two or more terminal devices of two or more users via the network NW. The single terminal device may be used by multiple users.

The livestock information management system 1 is a system for supporting management of livestock information mainly by a farmer. An operator of the livestock information management system 1 provides a service for supporting management of livestock information to a farmer using the system. The operator can provide services to multiple farmers. The livestock information management server 101 is managed by the operator of the livestock information management system 1, and the farmer uses the terminal device 201. The livestock information management server 101 may be managed by the farmer oneself.

The terminal device 201 is, for example, a device such as a PC, a smartphone, a tablet PC, and a mobile phone. The terminal device 201 may access a livestock information management server 101 via dedicated application software installed in the terminal. Alternatively, the terminal device 201 may access the livestock information management server 101 by using an operating environment (e.g., an application programming interface (API), and a platform) provided by the livestock information management server 101.

An input unit 211 is a device that can be operated by the user to input or select information, such as a keyboard, a mouse, or a touch pad. The input and selection of information are, for example, input by entering a numerical value, a character, or a symbol, selection of an item, and input for determining processing. Alternatively, a touch panel integrated with the display unit 212 such as a liquid crystal display or an organic EL display in a smartphone, a tablet, or a PC may be used. The input unit 211 may be a voice input device.

A display unit 212 is, for example, a display device that displays information or the like to the user. The display unit 212 may be a display device independent of the terminal device 201, or may be a display device such as a liquid crystal display or an organic EL display in a smartphone or a tablet.

The terminal device 201 may be integrated with the livestock information management server 101 without using the network NW

The livestock information management server 101 includes: a mother pig extraction unit 111, a production group management unit 112, a production group registration unit 113, an event result registration unit 114, a production group dynamics display unit 115, a prediction unit 116, and a notification unit 117. These units will be described in detail in descriptions of the embodiments.

The information storage unit 121 is a storage device which stores information to be used to perform management by the livestock information management system 1. As the information, information on livestock or a livestock herd, information on facilities such as farms and barns, information on the number and abilities of employees, information on materials such as feeds and drugs, and information on livestock industry operations such as events and statuses to be described later are stored. In FIG. 1, the information storage unit 121 is integrated with the livestock information management server 101, but may be a storage device physically separated from the livestock information management server 101. Information stored in the information storage unit 121 will now be described below.

The information storage unit 121 stores a mother pig identifier D11 and mother pig status information D12, as individual information on a mother pig. The information storage unit 121 further stores a production group identifier D21 and production group status information D22, as information on a production group. As information on a newborn baby group, a newborn baby group identifier D31 and newborn baby group status information D32 is included. The information storage unit 121 further stores breeding information D41 indicating a past breeding record. The information storage unit 121 further stores alert master information D51 which is a predetermined condition for notifying the user of a case satisfying a predetermined condition.

Mother pigs are sows reared for breeding in a pig farming industry, and also referred to as breeding mother pigs or breeding sows. Basically, the mother pigs are reared to give birth (childbirth) to piglets and to perform nursing. The mother pig identifier D11 is information for identifying each mother pig, and is an electronic identifier uniquely assigned to each actually existing mother pig in various forms such as a name, a number, and an ID of the mother pig. The actually existing mother pig is managed by assigning a physical identifier such as an ear tag, an IC tag, or a tattoo, on the premise that the physical identifier corresponds to an electronic mother pig identifier stored in the information storage unit 121.

The mother pig identifier D11 is stored in the information storage unit 121 in association with various pieces of information such as the mother pig status information D12, history information, an age, a parity, a gravidity, a breed, parent information, breeding pig information, and a birth record. The mother pig status information is information including a mother pig status indicating a rearing state of each mother pig. The mother pig status will be described in detail later. The history information is information indicating a timing which is date and time on which the mother pig status has transitioned.

The parity is a number counted as a record of normal childbirth in the past. The gravidity indicates a childbirth history including abnormalities such as miscarriage after pregnancy, and is a number counted at the time of breeding in the past.

The mother pig status information D12 includes information on the mother pig status. The mother pig status is stored, including, as a normal status where the status transitions to a desired rearing state for a farmer, bred, pregnant, in nursing, in weaning, and in estrus. The mother pig status is stored, including, as an abnormal status where the status transitions to an undesirable rearing state for the farmer, re-estrus, a delayed pregnancy test, non-pregnant, miscarriage, a delayed childbirth, delayed weaning, un-estrus/poor physical condition, delayed breeding, death, and disuse.

The production group is a set of multiple mother pigs, and is defined as a group on which the same breeding event is to be conducted at the same time. The production group may be one on which all events for breeding pigs are conducted consistently, i.e., a group of mother pigs on which events are conducted always together in a breeding cycle. Alternatively, the production group may be formed and reorganized for each event according to the mother pig status.

The production group formed and reorganized for each event can be formed as, for example, a breeding (mother pig) group formed according to the mother pig status after breeding, a nursing (mother pig) group formed according to the mother pig status during nursing, or a weaning (mother pig) group formed according to the mother pig status during weaning.

The production group identifier D21 is information for identifying the production group, and is an electronic identifier uniquely assigned to each production group in various forms such as a name, a number, or an ID.

The production group identifier D21 is stored in the information storage unit 121 in association with the mother pig identifier D11 belonging to the production group, the production group status information D22, history information, and other pieces of information.

The production group status information D22 is information including a production group status indicating a rearing state common to individual mother pigs belonging to the production group. Thus, the kinds of the status are similar to those of the mother pig status information D12. The history information is information indicating a timing which is date and time on which the production group status has transitioned.

Similarly to the mother pig status information D12, the production group status information D22 includes, as a normal status where the status transitions to a desired rearing state for a farmer, bred, pregnant, in nursing, in weaning, and in estrus. The production group status information D22 includes, as an abnormal status where the status transitions to an undesirable rearing state for the farmer, re-estrus, a delayed pregnancy test, non-pregnant, miscarriage, a delayed childbirth, delayed weaning, un-estrus/poor physical condition, delayed breeding, and death.

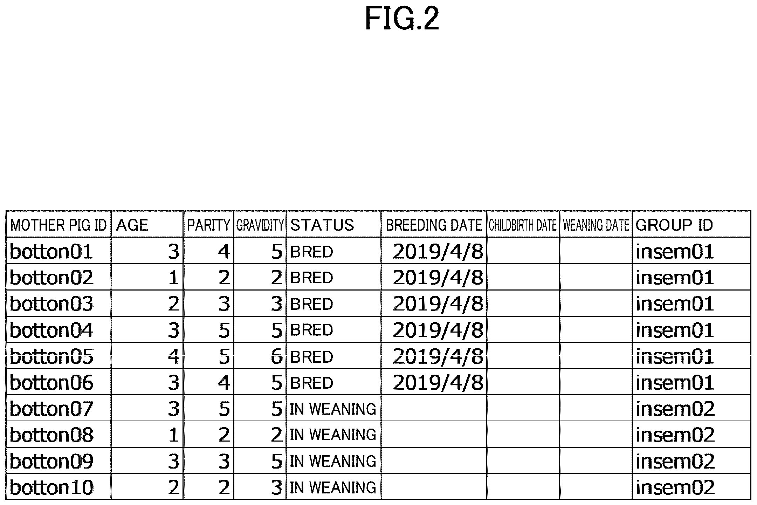

FIG. 2 is an example of the mother pig information stored in the information storage unit 121. For example, the mother pig ID is stored in association with an age, a parity, a gravidity, a status, a breeding date, a childbirth date, and a weaning date as the mother pig identifier D11 and a group ID as the production group identifier D21 of the production group to which the mother pigs belong. Multiple breeding dates, childbirth dates, and weaning dates may be stored according to a parity and a gravidity.

The newborn baby group is basically a piglet herd mainly forming a group to which a piglet herd born only of mother pigs belonging to a predetermined production group belongs. Thus, the production group and the newborn baby group are registered and stored in association with each other. That is, the production group and the newborn baby group are in a group relationship in which multiple mother pigs and multiple newborn babies are registered and stored in association with each other. The piglet herd belonging to the newborn baby group may be reorganized for each event at all times in view of an actual rearing status. The newborn baby group identifier D31 is information for identifying each newborn baby group, and is an electronic identifier uniquely assigned to each actually existing mother pig in various forms such as a name, a number, or an ID.

The newborn baby group identifier D31 is stored in the information storage unit 121 in association with the newborn baby group status information D32, history information, and other pieces of information.

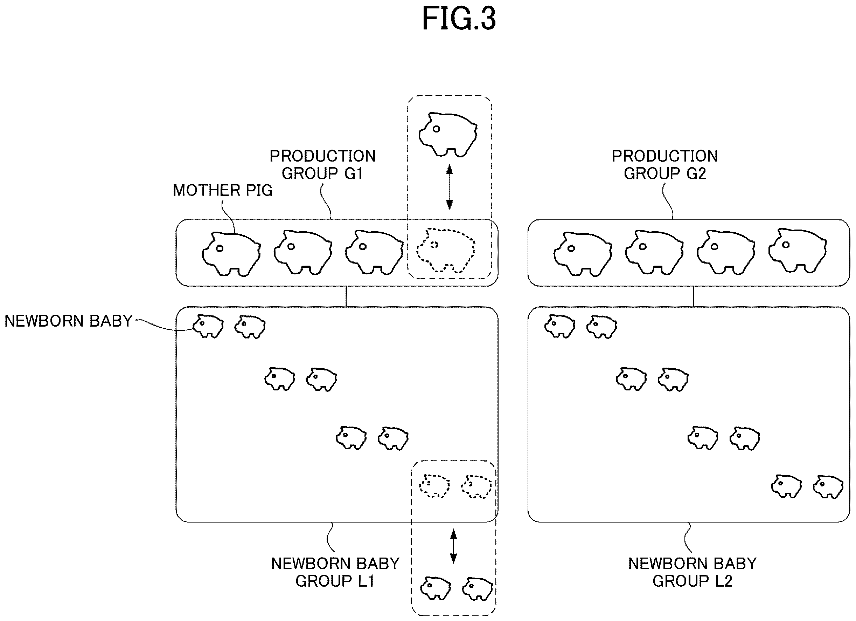

FIG. 3 illustrates a relationship between the production group and the newborn baby group. As described above, the production group is a set of mother pigs, and the newborn baby group is a piglet herd formed mainly of piglets born of mother pigs belonging to a predetermined production group. Multiple production groups are formed. As shown in FIG. 3, a production group G1 and a production group G2 are basically grouped so that their timings of performing an event can be controlled, and schedules thereof are managed. The groups fluctuate, and if a mother pig whose status has transitioned to the abnormal status due to a problem in physical condition or the like is in a group, the user basically determines adding or removing of the mother pig and gives an instruction, and the system stores and updates information on such a mother pig in response to the instruction. Similarly, the piglets belonging to the newborn baby group are also flexibly added or removed according to the user's instruction in response to the status transition.

The newborn baby group status information D32 is information including the newborn baby group status indicating the rearing state common to the piglet herd belonging to the newborn baby group. The history information is information indicating a timing which is date and time on which the status of the newborn baby group has transitioned.

The breeding information D41 is information on a breeding record such as past childbirth of mother pigs. For example, the breeding information D41 includes, as information, a past breeding record (histories of a childbirth date, a boar, the number of babies born, and other status transitions) of each mother pig, characteristics (a breed, an age, a parity, a gravidity, a breeding date (a breeding timing)) of the mother pig, and a newborn baby record (the weaning and shitting timings of a newborn baby, the shipping number of newborn babies, and the like).

The alert master information D51 is information in which conditions to be notified to the user as an alert by using the notification unit 117 are set and stored for information such as various numerical values, states, predictions, and records generated by the livestock information management system 1.

In the present embodiment, the event is a work to be conducted for rearing on mother pigs belonging to the production group or piglets belonging to the newborn baby group.

Examples of the event include the following. Breeding (including mating, artificial insemination (AI), embryo transfer (ET)) is a work to be conducted to get a mother pig pregnant. Direct mating between a boar and a sow without artificial insemination is also referred to as real mating. Acceptance is an event of accepting pigs such as breeding candidate pigs. Acclimatization is an event in which newly introduced pigs or pigs whose environment have changed, such as moving through a pig house, is acclimatized to the new environment. Feeding is an event of feeding individual pigs or a herd (a production group or a newborn baby group). Medication is an event of administering a drug to target individual pigs or a target herd (a production group or a newborn baby group). Moving is an event of moving target individual pigs or a target herd (a production group or a newborn baby group) to a different barn, room, or pen (a pig house, room, or pen). A hormone/physical treatment is an event of administering a hormone to a mother pig to promote estrus. The pregnancy test is an event of testing, by a veterinarian, whether or not a mother pig is pregnant using a diagnostic device or the like after mating. Vaccination is an event of administering a vaccine to target individual pigs or a target herd (a production group or a newborn baby group) Start of nursing is an event in which a mother pig starts nursing her babies after childbirth. Adoption (nursed pigling) is an event in which the number of piglets nursed by a mother pig after childbirth is controlled, and an event in which piglets are adopted or moved to be nursed by other mother pigs. Weaning is an event of moving piglets to a weaning house after the nursing period of the mother pig. Semen collection is an event of collecting semen from boars. Death determination is an event of determining that target livestock or a target livestock herd (a production group or a newborn baby group) has died. Shipping is an event of shipping target individual pigs or a target herd (a production group or a newborn baby group). Disuse is an event of discarding pigs that are no longer worth rearing among target individual pigs or a target herd (a production group or a newborn baby group). All-out is an event of cleaning and disinfecting facilities, such as a pig house, which have become empty after shipping. Candidate pig sorting is an event of sorting growing pigs to be mother pig candidates among piglets.

The events are not limited to the works described above, and may include other items.

In the present embodiment, the status indicates a rearing state of livestock, which transitions according to the result of the event conducted. The status is the result of the event conducted, and may be a number of different results. The livestock information management system 1 does not determine the status to which the current status is to transition to or the result itself of the event. The status transition is determined in the order in which the event is to be conducted, which is defined in event schedule master information in accordance with the result of information selected and input by the user. Conduct of the event is stored in an information storage unit 121 by selection and input performed by the user with the input unit 211 of the terminal device 201 shown in FIG. 1. When the conduct of the event is registered, the status transitions as a result of the information selected and input. The status transition and the timing thereof are stored in the information storage unit 121 as status information and history information.

Among the statuses, in particular, a state scheduled for rearing livestock is referred to as a normal status. On the other hand, a state which is expected in rearing of livestock but is not preferable for a farmer is referred to as an abnormal status. Each status is included and stored, as qualitative data, in the mother pig status information D12, the production group status information D22, and the newborn baby group status information D32 in the information storage unit 121.

The normal status for the state of livestock in the category of female breeding livestock indicates, for example, states of being bred, in nursing, in weaning, pregnant, and in estrus.

The abnormal status for the state of livestock in the category of female breeding livestock indicates, for example, states of delayed childbirth, delayed weaning, delayed breeding, re-estrus, miscarriage, disease, death, a delayed pregnancy test, non-pregnant, and un-estrus/poor physical condition.

The following describes the statuses. Being bred indicates a state in which a mother pig has been bred, i.e., mated. Being pregnant indicates a state in which a fertilized egg has been imbedded and confirmed by a pregnancy test. Being in nursing is a state in which a mother pig is nursing her baby after childbirth. Being in weaning is a state from the end of the nursing period of the mother pig to confirmation of the return of estrus. Being in estrus is a state in which the weaning phase has ended and normal return to estrus has been confirmed by an estrus/physical condition check. The re-estrus is a state in which a mother pig has failed to be mated and is in re-estrus behavior. The delayed pregnancy test is a state in which a pregnancy test is delayed. The non-pregnant is a state in which it has been confirmed by a pregnancy test that a mother pig is not pregnant. The miscarriage is a state in which a mother pig was pregnant but had a miscarriage without giving a birth. The disease is a state in which a rearing pig is affected with a disease. The delayed childbirth is a state in which the childbirth of a pregnant mother pig is delayed. The delayed weaning is a state in which weaning is delayed. The delayed breeding is a state in which breeding is delayed. The death is a state in which the livestock has died.

The statuses are not limited to the states described above, and may include other items.

The event information includes information on events which are works conducted for rearing the livestock described above.

The event schedule master information includes a livestock status transition flow indicating an order and a route in which livestock in a certain status after a certain event has been conducted should next transition to. Specifically, the event schedule master information includes a normal status transition flow indicating a route in which the status of the livestock transitions to the normal status scheduled for rearing the livestock, and an abnormal status transition flow indicating a route in which the status of the livestock transitions to the abnormal status which is a state expected in rearing of livestock but is not preferable and a route in which the status returns from the abnormal status to the normal status.

Further, the event schedule master information includes information defining a timing at which next event is to be conducted after a certain event has been conducted. More specifically, the event schedule master information includes event interval information indicating an interval between events.

### <Production Group Management Function>

### (First Embodiment)

A first embodiment will now be described below.

The mother pig extraction unit 111 illustrated in FIG. 1 has a function of extracting multiple mother pigs in the same mother pig status by using the mother pig status information.

The production group registration unit 113 has a function of associating the mother pigs extracted by the mother pig extraction unit 111 with a mother pig identifier and assigning, to the mother pigs, a production group identifier for identifying the mother pigs as a production group on which the same breeding event is to be conducted at the same timing.

The event result registration unit 114 has a function of receiving and registering information on results of events conducted by the user on mother pigs or piglets. Based on the registered information, the status of the mother pigs, the piglets, or a group thereof transitions.

The mother pig extraction unit 111 may display, as candidates of mother pigs which are to belong to the production group, extracted candidates on a selection screen so that the user can perform selection from the candidates.

The mother pig extraction unit 111 may extract, as a group candidate, mother pigs whose timing at which the mother pig status has transitioned satisfies a predetermined condition by using the history information.

The production group management unit 112 has a function of excluding, when there is a mother pig whose mother pig status has transitioned to the abnormal status, the mother pig from the production group to which the mother pig belongs, by using the mother pig status information.

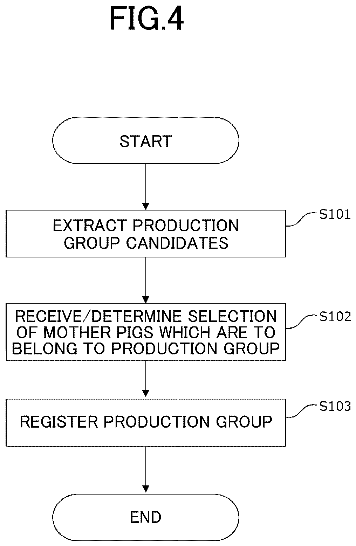

The process flow of the first embodiment will now be described below. FIG. 4 is a flowchart illustrating the flow of the processes of the first embodiment.

In Step S101, the mother pig extraction unit 111 refers to the mother pig identifier D11 stored in the information storage unit 121 for mother pigs which are candidates of mother pigs which are to belong to the production group, and extracts a mother pig identifier of corresponding mother pigs according to the predetermined condition.

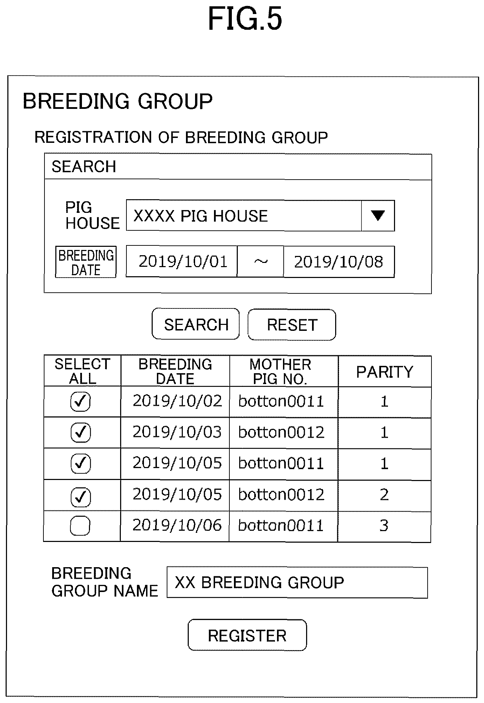

FIG. 5 is an example of a screen displayed on the display unit 212 for the mother pigs extracted as the candidates of the mother pigs which are to belong to the production group. As will be described later, FIG. 5 is also an example of a screen displaying options for the user to determine the mother pigs which are to belong to the production group.

For example, the production group may be registered for each event as in "REGISTRATION OF BREEDING GROUP" in FIG. 5. This screen is an example of a screen for registering a breeding (mother pig) group. The mother pig extraction unit 111 refers to the mother pig status information stored in the information storage unit 121, and extracts a mother pig identifier for mother pigs in a predetermined mother pig status (in FIG. 5, bred). Then, the extracted mother pig identifier for the mother pigs is presented to the user. In addition to the mother pig identifier, related information such as a status transition date (a breeding date in FIG. 5) and a parity associated therewith may also be presented to the user. Such presentation to the user can be performed using the display unit 212 of the terminal device 201.

The mother pig extraction unit 111 may extract the candidates of the mother pigs which are to belong to the production group by referring to the history regarding the timing when the mother pig status has transitioned and using a predetermined a predetermined status transition timing as a condition. For example, as displayed on the terminal screen in FIG. 5, a breeding group is created with a breeding date as a starting point. The user can input the dates which are the starting and end points of breeding by using the input unit 211 of the terminal device 201, and mother pigs whose status has transitioned to the mother pig status of being in breeding during a predetermined breeding period can be extracted as candidates.

In Step S102, mother pigs which are to belong to the production group among the candidates of the mother pigs which are to belong to the production group, extracted by the mother pig extraction unit 111, are selected and received, or determined.

As shown in FIG. 5, the mother pig candidates shown on the display unit 212 by the mother pig extraction unit 111 are presented in a selectable manner. The user thus may determine the mother pig candidates which are to belong to the production group by using the input unit 211. For example, the user checks a check box corresponding to a mother pig which is to belong to the production group, and clicks or taps a registration button.

Alternatively, the mother pig extraction unit 111 may automatically determine the mother pigs which are to belong to the production group based on a predetermined extraction condition (e.g., mother pigs bred within a predetermined breeding period). In any case, in Step S102, the mother pigs which are to belong to the production group are determined.

In Step S103, the production group registration unit 113 registers the mother pigs determined/selected in Step S102 to the production group. Specifically, the production group identifier is stored in association with the mother pig identifier stored in the information storage unit 121.

In this manner, the production group can be registered as a set of multiple mother pigs on which the same breeding event is to be conducted at the same time. For a mother pig herd registered as a production group, when the user inputs, as information, results of an event conducted thereafter to the livestock information management system 1, the user can access the mother pig identifiers of all the mother pigs belonging to the production group based on the production group identifier by using the event result registration unit 114 and input, for all the mother pig identifiers at once, the results of the event conducted to store the results. This reduces an input load on the user.

In addition, various situations can be grasped by using information on the production group registered. This will be described later.

The information on the production group registered can be edited by the production group management unit 112 each time. For example, when one of mother pigs belonging to a predetermined production group is affected by a disease and the same breeding event cannot be conducted on the mother pigs at the same timing, a mother pig identifier corresponding to the mother pig affected by the disease can be excluded from the predetermined production group. Specifically, the mother pig can be excluded by deleting the production group identifier from information on the mother pig to be excluded from the production group or updating the information to not-belonging, for example. The production group management unit 112 can edit the production group information in response to an editing instruction from the user.

Alternatively, the production group management unit 112 may extract a mother pig whose status has transitioned to the abnormal status, and automatically perform the processing of excluding the mother pig from the production group. For example, the production group management unit 112 refers to mother pig status information on mother pigs belonging to a predetermined production group in accordance with a predetermined exclusion condition. Then, a mother pig which is in the abnormal status and satisfies the condition may be extracted, and the processing of excluding the mother pig from the production group may be performed. Before automatic exclusion, the production group management unit 112 may display, on the display unit 212 of the terminal device 201, a screen for requesting the user to determine whether or not to the mother pig is to be excluded from the production group, and may perform the processing of excluding the mother pig from the production group when the user accepts exclusion by using the input unit 211.

As described above, the livestock information management system according to the first embodiment of the present disclosure includes: the mother pig extraction unit 111 configured to extract mother pigs in the same mother pig status by using the mother pig status information; and the production group registration unit 113 configured to associate the mother pigs extracted by the mother pig extraction unit 111 with a mother pig identifier and assigning, to the mother pigs, a production group identifier for identifying the mother pigs as a production group on which the same breeding event is to be conducted at the same timing. Thus, the production group can be registered as a set of multiple mother pigs on which the same breeding event is to be conducted at the same time. For a mother pig herd registered as a production group, when the user inputs results of an event conducted thereafter to the livestock information management system 1, the user can input, for all the mother pigs belonging to the production group at once, the results of the event conducted based on a production group identifier by using the event result registration unit 114. This can reduce an input load on the user. In addition, the status of the information on the mother pig herd registered as the production group can be sequentially grasped. In other words, mother pig herds which are important in a livestock industry business can be managed while sequentially grasping the statuses thereof.

The mother pig extraction unit 111 is configured to display, as candidates of mother pigs which are to belong to a production group, extracted candidates on a selection screen so that the user can perform selection from the candidates. This can reduce the load of creation of the production group on the user.

The mother pig extraction unit 111 is configured to extract, as a group candidate, mother pigs whose timing at which a mother pig status has transitioned satisfies a predetermined condition by using the history information. This can reduce the load of creation of the production group on the user.

### <Newborn Baby Group Management Function>

### (Second Embodiment)

A second embodiment will now be described below.

The production group registration unit 113 illustrated in FIG. 1 has a function of registering a newborn baby group to be associated with a production group which is a set of mother pigs and storing the newborn baby group in the information storage unit 121.

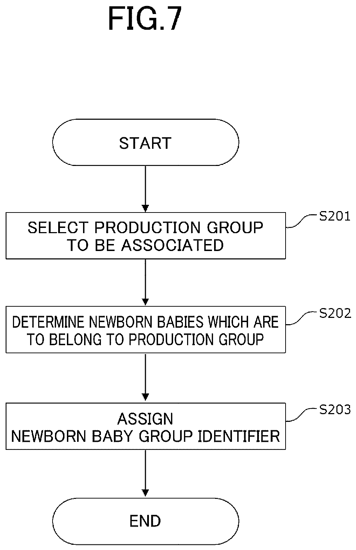

The process flow of the second embodiment will now be described below. FIG. 7 is a flowchart illustrating the flow of the processes of the second embodiment.

In Step S201, the production group registration unit 113 determines the production group to be associated with the newborn baby group in response to a selection instruction by the user.

In Step S202, the production group registration unit 113 determines newborn babies which are to belong to the newborn baby group. Candidates for piglets which are to belong to the newborn baby group are, for example, a piglet herd which has been just born and has not been group-registered yet. The number of piglets registered in the piglet herd may be one.

In Step S203, a newborn baby group identifier is assigned to the piglet herd determined to belong to the newborn baby group by the user's instruction, and is stored in the information storage unit 121.

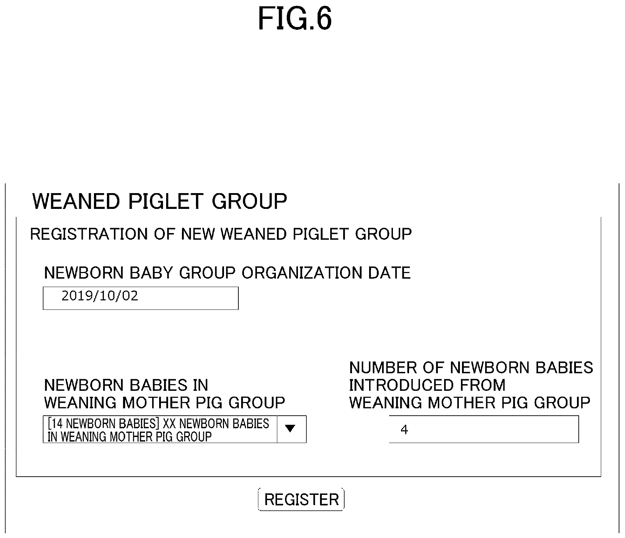

FIG. 6 shows an example of a screen. This screen is a screen for registering a weaned piglet group, which is one of newborn baby groups, as a new weaned piglet group registration. The screen is configured such that the user selects a production group (a weaning mother pig group on this screen) to be associated with the new weaned piglet group, and four piglets are selected from 14 piglets actually born of the weaning mother pig group and registered as the new weaned piglet group.

As described above, in the livestock information management system according to the second embodiment of the present disclosure, the production group registration unit 113 is configured to register a newborn baby group to be associated with a production group which is a set of mother pigs and store the newborn baby group in the information storage unit 121. Thus, parent pigs and piglets can be grasped in association with each other. Further, as will be described later, the transition of the status until piglets are shipped can be grasped by using the associated data.

### <Dynamics Display Function>

### (Third Embodiment)

A third embodiment will now be described below.

The production group dynamics display unit 115 illustrated in FIG. 1 has a function of calculating actual values of the number of mother pigs belonging to each of multiple production groups at multiple arbitrary timings by using the mother pig status information and the production group identifier and displaying, for each production group, a time series of the number of mother pigs belonging to the production group.

The production group dynamics display unit 115 further has a function of calculating the number of mother pigs belonging to each of multiple production groups whose statuses have transitioned to the abnormal status at multiple arbitrary timings by using the mother pig status information and the production group identifier and displaying a time series of the number of the mother pigs for each production group.

The prediction unit 116 has a function of predicting predicted values of the number of mother pigs belonging to the production group at multiple arbitrary timings in accordance with the breeding timing and the parity and/or the gravidity of the mother pigs belonging to the production group by using the breeding information.

The prediction unit 116 further has a function of predicting predicted values of the number of babies born in the production group or scheduled childbirth dates in the production group in accordance with the breeding timing and the parity and/or the gravidity of the mother pigs belonging to the production group by using the breeding information.

The production group dynamics display unit 115 has a function of calculating actual values of the number of mother pigs belonging to each of multiple production groups at multiple arbitrary timings by using the mother pig status information and the production group identifier, displaying, for each production group, a time series of the number of mother pigs belonging to the production group, and displaying predicted values predicted by the prediction unit 116 for values whose actual values of the number of mother pigs belonging to each of the production groups at the arbitrary timings are missing.

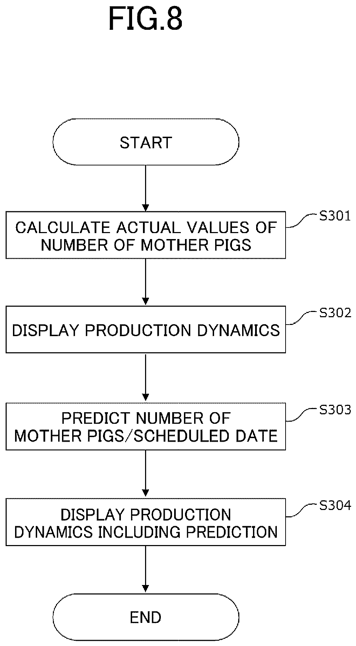

The process flow of the third embodiment will now be described below. FIG. 8 is a flowchart illustrating the flow of the processes of the third embodiment.

In Step S301, the production group dynamics display unit 115 refers to the mother pig identifier D11 and the mother pig status information D12 and calculates actual values of the number of mother pigs in a predetermined production group at multiple arbitrary timings, in order to visually present the dynamics of multiple production groups to the user. Specifically, a periodic time series such as every one week or every ten days or an arbitrary time series is provided, and the number of mother pigs belonging to the production group as mother pigs in a predetermined status such as bred at each timing of the time series is calculated. Hereinafter, for the sake of simplicity, the "time series of the number of mother pigs belonging to the production group at the multiple arbitrary timings" is also referred to as "dynamics." In particular, a time series of the number of mother pigs belonging to the production group at the multiple arbitrary timings for a production group which is a set of mother pigs which can maintain their normal statuses such as bred to continue breeding activities is referred to as production dynamics, and the number of mother pigs dropped or excluded from the breeding activities due to transition to the abnormal status such as miscarriage at the multiple arbitrary timings is also referred to as abnormal dynamics.

In Step S302, a time series of the calculated actual values of the number of mother pigs which are active at the multiple arbitrary timings is displayed on the display unit 212 of the terminal device 201, for example.

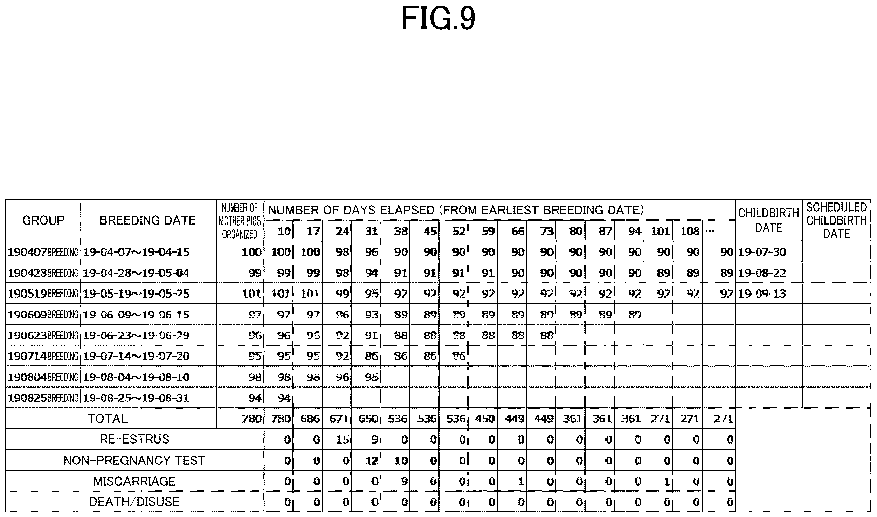

FIG. 9 is an example of a screen displayed on the display unit 212 of the terminal device 201 by the production group dynamics display unit 115. As illustrated in FIG. 9, for each of multiple production groups, production dynamics are shown every one week based on a breeding date. The production dynamics shown by the production group dynamics display unit 115 indicate the number of mother pigs in active in every number of days elapsed from the earliest breeding date (the earliest date of breeding) as a starting date in each group for eight production groups (breeding (mother pig) groups) different from each other in a breeding period. FIG. 9 further illustrates, for each group, an initial number of mother pigs organized. FIG. 9 illustrates a state where as the number of days elapsed increases, the number of mother pigs in active decreases from the initial number of mother pigs organized because some of the mother pigs have transitioned to the abnormal status such as re-estrus, a non-pregnancy test, or miscarriage. The user can recognize the number of mother pigs in active through the visualized information. This can support improvement in a livestock industry business efficiency.

Similarly for mother pigs whose statuses have transitioned to the abnormal status, the number of these mother pigs is also shown in a time series as the abnormal dynamics together with the breakdown of each abnormal status. As a result, the user can recognize when the abnormality occurs through the visualized information. This can support improvement in the livestock industry business efficiency.

For the transition to the abnormal status, information stored is updated according to the result of the status transition input by the user as described above, the updated information is reflected on the contents displayed by the production group dynamics display unit 115.

As described above, the production group dynamics display unit 115 can display, as the production dynamics, the actual values of the number of mother pigs in active in each production group based on the actual values of the status transition. Furthermore, the following describes the process flow in which the prediction unit 116 predicts future events for which actual event results and status transition have not occurred yet, and displays these events as dynamics.

In Step S303, the prediction unit 116 predicts predicted values of the number of mother pigs belonging to each production group at multiple arbitrary timings based on past breeding information D41 on the mother pigs. The predicted values are, for example, a scheduled childbirth date and the number of babies born (the number of childbirths) in addition to the number of mother pigs in active at each timing. The number of babies born includes those predicted as a numerical value range. Similarly, the scheduled childbirth date includes not only a date limited to one day, but also a date predicted as a period, i.e., the interval of time.

The breeding information D41 includes a past breeding record (histories of a childbirth date, the number of babies born, and other status transitions) of each mother pig and characteristics (a breed, an age, a parity, a gravidity, and a breeding date (a breeding timing)) of such a mother pig as described above. The prediction unit 116 predicts the number of mother pigs in active, the scheduled childbirth date, the number of babies born, and the like in each production group at each timing on the basis of the pieces of information in accordance with the number of mother pigs (the number of mother pigs organized) belonging to each production group currently organized and the characteristics thereof. For example, the pregnancy rate of pigs varies depending on a season, such as heat stress. Thus, a breeding timing such as a breeding date is an important explanatory variable. Further, factors such as whether or not the mother pigs are primiparous mother pigs or parous mother pigs and the parity/gravidity are also large factors that influence the pregnancy rate, and are important explanatory variables. The prediction unit 116 makes prediction based on these characteristics of the mother pigs belonging to each production group. As a prediction model used for this purpose, a decision tree, a regression model, a deep neural network, or ensemble learning thereof can be used.

The predicted values predicted by the prediction unit 116 are not limited to use for displaying the predicted values in a time series by the production group dynamics display unit 115 to be described later, and may be displayed or notified to the user as independently useful information. The user can recognize the future status of the mother pigs in active by predicting a predicted value of the number of mother pigs belonging to each production group at a predetermined arbitrary timing. In addition, by predicting the predicted values of the number of babies born or the scheduled childbirth date in the production group, the user can recognize livestock products to be obtained in the future and works to be generated in the future, and can further consider resource planning.

In Step S304, the production group dynamics display unit 115 displays, e.g., the predicted values predicted by the prediction unit 116 instead of blanks (blank timings) where the actual values of the production dynamics shown by using the display unit 212 do not exist.

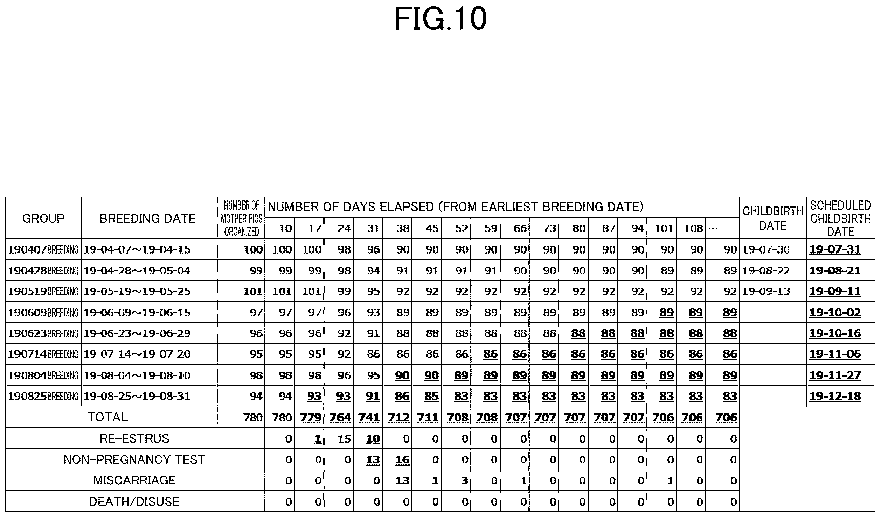

FIG. 10 is an example of a screen displaying, e.g., the predicted values predicted by the prediction unit 116 instead of the blanks where the actual values do not exist, in addition to the actual values of the production dynamics illustrated in FIG. 9. In FIG. 10, portions where the predicted values and the like are displayed as differences from FIG. 9 are indicated by bold and underlined numbers and symbols. For example, in a breeding (mother pig) group indicated as "190825 BREEDING," actual values after a lapse of 17 days from the earliest breeding date do not exist, but numerical values predicted by the prediction unit 116 are displayed. This can estimate how many mother pigs are in active in the future. Further, for the scheduled childbirth date, predicted childbirth dates predicted by the prediction unit 116 are displayed. This can estimate when a childbirth work occurs. Further, virtual total numbers of mother pigs by mixing the actual values and the predicted values are shown in the column of "TOTAL." The number of mother pigs in active in the future can be estimated from the virtual total number of mother pigs. The virtual total numbers of mother pigs are effective for estimating an effective utilization rate of a facility such as a pig house in the future and the shipping number of porkers. In this manner, the user can recognize the number of mother pigs in active in the future through the visualized information. This can provide future prospects and can support improvement in the livestock industry business efficiency.

As described above, in the livestock information management system according to the third embodiment of the present disclosure, the production group dynamics display unit 115 further calculates the actual values of the number of mother pigs belonging to each of the multiple production groups at the multiple arbitrary timings by using the mother pig status information and the production group identifier, and displays, for each production group, the time series of the number of the mother pigs belonging to the production group. As a result, the user can recognize the number of mother pigs in active through the visualized information. This can support improvement in the livestock industry business efficiency.

The production group dynamics display unit 115 further calculates the number of mother pigs belonging to each of the multiple production groups whose statuses have transitioned to the abnormal status at the multiple arbitrary timings by using the mother pig status information and the production group identifier, and displays the time series of the number of the mother pigs for each production group. As a result, the user can recognize when the abnormality occurs through the visualized information. This can support improvement in the livestock industry business efficiency.

The prediction unit 116 predicts the predicted values of the number of mother pigs belonging to the production group at the multiple arbitrary timings in accordance with the breeding timing and the parity and/or the gravidity of the mother pigs belonging to the production group by using the breeding information. This allows the user to recognize the future status of the mother pigs in active.

The prediction unit 116 further predicts predicted values of the number of babies born in the production group or scheduled childbirth dates in the production group in accordance with the breeding timing and the parity and/or the gravidity of the mother pigs belonging to the production group by using the breeding information. This allows the user to recognize livestock products to be obtained in the future and works to be generated in the future.

The production group dynamics display unit 115 calculates actual values of the numbers of mother pigs belonging to each of multiple production groups at multiple arbitrary timings by using the mother pig status information and the production group identifier, displays, for each production group, a time series of the numbers of mother pigs belonging to the production group, and displays predicted values predicted by the prediction unit 116 for portions whose actual values of the number of mother pigs belonging to each of the production groups at the multiple arbitrary timings are missing. The user thus can recognize the predicted values through the visualized information. This can provide future prospects and can support improvement in the livestock industry business efficiency.

### <Alert Notification Function>

### (Fourth Embodiment)

A fourth embodiment will now be described below.

The notification unit 117 shown FIG. 1 has a function of calculating the number of mother pigs in each of multiple production groups whose statuses have transitioned to the abnormal status at an arbitrary timing by using the mother pig status information and the production group identifier and making notification to the user when the number of the mother pigs satisfies a predetermined condition.

The notification unit 117 further has a function of making notification to the user when a predicted value of the number of mother pigs belonging to the production group satisfies a predetermined condition.

The notification unit 117 further has a function of making notification to the user when a predicted value of the number of babies born satisfies a predetermined condition.

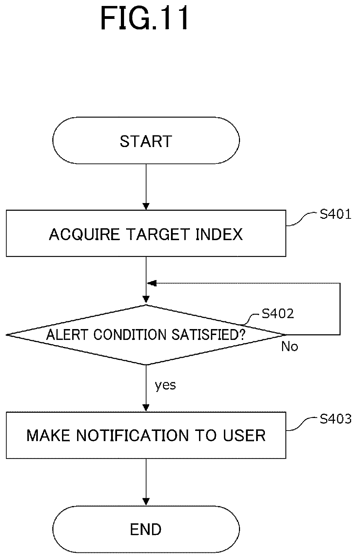

The process flow of the fourth embodiment will now be described below. FIG. 11 is a flowchart illustrating the flow of the processes of the fourth embodiment.

In Step S401, the notification unit 117 acquires a predetermined target index set in advance. The predetermined target index specifically include, for example, the presence or absence of transition of the status of a mother pig to the abnormal status, the number of mother pigs whose statuses have transitioned to the abnormal status, the number of mother pigs belonging to a predetermined production group, the predicted number of mother pigs predicted as belonging to a predetermined production group at a predetermined timing by the prediction unit 116, and the predicted number of babies born predicted as being born of mother pigs belonging to a predetermined production group at a predetermined timing by the prediction unit 116. In addition, the target index can be set by combining information stored in the information storage unit 121, a predicted value predicted by the prediction unit 116, and the like.

In Step S402, the notification unit 117 determines whether or not the acquired target index is to be notified to the user, by comparing the target index with an alert condition. The alert condition is stored in the alert master information D51 of the information storage unit 121. The alert condition can be flexibly set to a condition where the target index exceeds a reference numerical value, a condition where the target index falls below the reference numerical value, a condition where the target index is the same as the reference numerical value, or a combination thereof.

In Step S403, if the target index satisfies the alert condition (yes), the notification unit 117 makes notification to the user, and the process ends. If the target index does not satisfy the alert condition (no), the notification unit 117 does not make notification.

As specific notification means, an alert may be displayed on a screen by using the display unit 212 of the terminal device 201, or may be notified by other means such as transmission of an e-mail to the user.

As described above, in the livestock information management system according to the fourth embodiment of the present disclosure, the notification unit 117 has a function of calculating the number of mother pigs belonging to each of production groups whose statuses have transitioned to the abnormal status at an arbitrary timing by using the mother pig status information and the production group identifier and making notification to the user when the number of mother pigs satisfies a predetermined condition. This allows the user to early recognize the status and early consider to improve livestock management.

The notification unit 117 allows the user to early recognize the status and early consider to improve livestock management when a predicted value of the number of mother pigs belonging to the production group satisfies a predetermined condition.

The notification unit 117 allows the user to early recognize the status and early consider to improve livestock management when a predicted value of the number of babies born satisfies a predetermined condition.

### (Program)

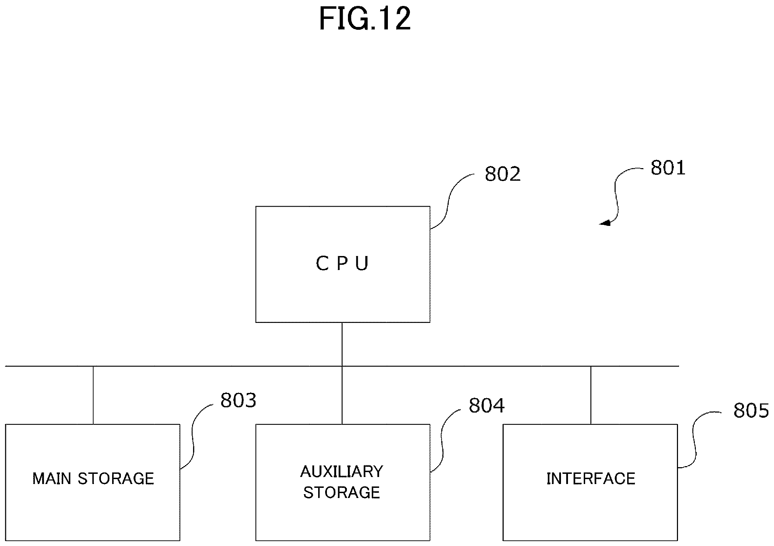

FIG. 12 is a schematic block diagram showing the configuration of a computer 801. The computer 801 includes a CPU 802, a main storage 803, an auxiliary storage 804, and an interface 805.

Here, a program for realizing each function constituting the livestock information management server 101 according to the embodiment will be described in detail.

The livestock information management server 101 is mounted on the computer 801. The operation of each component of the livestock information management server 101 is stored in the auxiliary storage 804 in the form of a program. The CPU 802 reads the program from the auxiliary storage 804, expands the program to the main storage 803, and executes the processes according to the program. In addition, the CPU 802 allocates storage areas corresponding to the above-described storage units in the main storage 803 according to the program.

Specifically, the program causes the computer 801 to execute: a mother pig extraction step of extracting, by the mother pig extraction unit 111, multiple mother pigs in the same mother pig status by using mother pig statuses indicating rearing states of the mother pigs transitioning by conducting a breeding event on the mother pigs and a history related to timings at which the mother pig statuses have transitioned; and a production group registration step of assigning, by the production group registration unit 113, a production group identifier for identifying the extracted mother pigs as a production group on which the same breeding event is to be conducted at the same timing.

The auxiliary storage 804 is an example of a non-transitory tangible medium. Other examples of the non-transitory tangible medium include magnetic disks, magneto-optical disks, CD-ROMs, DVD-ROMs, and semiconductor memories, to be connected via the interface 805. Alternatively, if the program is distributed to the computer 801 via the network NW, the computer 801 receiving the distribution may expand the program to the main storage 803, and execute the processes.

The program may realize some of the above-described functions. The program may also be a so-called differential file (differential program) that realizes the above-described functions in combination with other programs which have been stored in the auxiliary storage 804.

Although some embodiments of the present disclosure have been described above, these embodiments can be implemented in various other forms, and various omissions, substitutions, and changes can be made without departing from the spirit of the present disclosure. These embodiments and variations thereof are included in the scope and spirit of the present disclosure, and are also included in the disclosure described in the claims and the equivalent scope thereof.

### DESCRIPTION OF REFERENCE CHARACTERS

- 1: Livestock Information Management System
- 101: Livestock Information Management Server
- 111: Mother Pig Extraction Unit
- 112: Production Group Management Unit
- 113: Production Group Registration Unit
- 114: Event Result Registration Unit

- 115: Production Group Dynamics Display Unit
- 116: Prediction Unit
- 117: Notification Unit
- 121: Information Storage Unit
- 201: Terminal Unit
- 211: Input Unit
- 212: Display Unit
- 213: Display Screen
- 801: Computer
- 802: CPU
- 803: Main Storage
- 804: Auxiliary Storage
- 805: Interface

## Claims

1. A livestock information management system for managing breeding of multiple mother pigs, the system **characterized by** comprising:
an information storage unit configured to store mother pig identifiers for identifying the respective mother pigs and mother pig status information including mother pig statuses indicating rearing states of the mother pigs whose statuses transition by conducting a breeding event on the mother pigs identified by the mother pig identifiers and history information indicating timings at which the mother pig statuses have transitioned, in association with each other;
a mother pig extraction unit configured to extract multiple mother pig identifiers in an identical mother pig status by using the mother pig status information; and
a production group registration unit configured to assign a production group identifier in association with the mother pig identifiers extracted by the mother pig extraction unit, the production group identifier identifying the mother pigs as a production group on which an identical breeding event is to be conducted at an identical timing.

2. The livestock information management system of claim 1, wherein
the mother pig statuses include at least any one of being bred, in nursing, or in weaning.

3. The livestock information management system of claim 2, wherein
the production group includes at least any one of a breeding mother pig group, a nursing mother pig group, or a weaning mother pig group created in accordance with the mother pig statuses.

4. The livestock information management system of any one of claims 1 to 3, wherein
the mother pig extraction unit is configured to display, as candidates of mother pigs which are to belong to the production group, extracted candidates on a selection screen so that a user can perform selection from the candidates.

5. The livestock information management system of any one of claims 1 to 3, wherein
the mother pig extraction unit extracts mother pig identifiers of mother pigs for which timings when the mother pig statuses have transitioned are under a predetermined condition as group candidates which are candidates of mother pigs which are to belong to the production group, by using the history information.

6. The livestock information management system of any one of claims 1 to 3, wherein
the information storage unit further stores a newborn baby group identifier for identifying a piglet herd born of the mother pigs belonging to the production group, the newborn baby group identifier being associated with the production group identifier, and a newborn baby group status which is a rearing state of the piglet herd whose status transitions by conducting a rearing event on the piglet herd identified by the newborn baby group identifier, the newborn baby group status being associated with the newborn baby group identifier.

7. The livestock information management system of any one of claims 1 to 3, the mother pig status information further including an abnormal status indicating an abnormal state which occurs in rearing of the mother pigs, the system further comprising:
a production group management unit configured to exclude, when there is a mother pig whose mother pig status has transitioned to the abnormal status, the mother pig from a production group to which the mother pig belongs, by using the mother pig status information and the history information.

8. The livestock information management system of claim 7, wherein
the abnormal status includes at least any one of re-estrus, non-pregnant, miscarriage, death, or disuse.

9. The livestock information management system of any one of claims 1 to 3, further comprising:
a production group dynamics display unit configured to calculate actual values of the number of mother pigs belonging to each of multiple production groups at multiple arbitrary timings by using the mother pig status information and the production group identifier and display a time series of the numbers of mother pigs for each production group.

10. The livestock information management system of any one of claims 1 to 3, the mother pig status information further including an abnormal status indicating an abnormal state which occurs in rearing of the mother pigs, the system further comprising:
a production group dynamics display unit configured to calculate the number of mother pigs belonging to each of multiple production groups whose statuses have transitioned to the abnormal status at multiple arbitrary timings by using the mother pig status information and the production group identifier and display a time series of the number of mother pigs for each production group.

11. The livestock information management system of any one of claims 1 to 3, the information storage unit further storing breeding information associated with a breeding timing, a parity and/or a gravidity, and a history of transition to the abnormal status, which are past mother pig breeding results, the system further comprising:
a prediction unit configured to predict predicted values of the number of mother pigs belonging to the production group at multiple arbitrary timings in accordance with the breeding timing and the parity and/or the gravidity of the mother pigs belonging to the production group by using the breeding information.

12. The livestock information management system of claim 11, wherein
the breeding information further includes the number of babies born associated with the breeding timing and the parity and/or the gravidity which are the past mother pig breeding results, and
the prediction unit predicts predicted values of the number of babies born in the production group or scheduled childbirth dates in the production group in accordance with the breeding timing and the parity and/or the gravidity of the mother pigs belonging to the production group by using the breeding information.

13. The livestock information management system of claim 11, further comprising:
a production group dynamics display unit configured to calculate actual values of the number of mother pigs belonging to each of multiple production groups at multiple arbitrary timings by using the mother pig status information and the production group identifier, display a time series of the number of the mother pigs for each production group, and display the predicted values predicted by the prediction unit for portions whose actual values of the number of mother pigs belonging to each of the multiple production groups at the multiple arbitrary timings are missing.

14. The livestock information management system of any one of claims 1 to 3, the information storage unit further storing, as the mother pig status information, an abnormal status indicating an abnormal state which occurs in rearing of the mother pigs, and further storing alert master information including a predetermined condition under which a user is to be notified of the number of mother pigs whose statuses have transitioned to the abnormal status, the system further comprising:
a notification unit configured to calculate the number of mother pigs belonging to each of multiple production groups whose statuses have transitioned to the abnormal status at an arbitrary timing by using the mother pig status information and the production group identifier and make notification to the user when the number of the mother pigs satisfies the predetermined condition.

15. The livestock information management system of claim 11, the information storage unit further storing alert master information including a predetermined condition under which the user is to be notified of the predicted values of the number of mother pigs, the system further comprising:
a notification unit configured to make notification to the user when the predicted values of the number of mother pigs satisfy the predetermined condition.

16. The livestock information management system of claim 12, the information storage unit further storing alert master information including a predetermined condition under which the user is to be notified of the predicted values of the number of babies born, the system further comprising:
a notification unit configured to make notification to the user when the predicted values of the number of babies born satisfy the predetermined condition.

17. A livestock information management server for managing breeding of multiple mother pigs, the server **characterized by** comprising:
an information storage unit configured to store mother pig identifiers for identifying the respective mother pigs and mother pig status information including mother pig statuses indicating rearing states of the mother pigs whose statuses transition by conducting a breeding event on the mother pigs identified by the mother pig identifiers and a history indicating timings at which the mother pig statuses have transitioned, in association with each other;
a mother pig extraction unit configured to extract multiple mother pigs in an identical mother pig status by using the mother pig status information; and
a production group registration unit configured to assign a production group identifier in association with the mother pig identifiers extracted by the mother pig extraction unit, the production group identifier identifying the mother pigs as a production group on which an identical breeding event is to be conducted at an identical timing.

18. A livestock information management method for managing breeding of multiple mother pigs by a computer, the method **characterized by** comprising:
a mother pig extraction step of extracting, by a mother pig extraction unit, multiple mother pigs in an identical mother pig status by using mother pig identifiers for identifying the respective mother pigs, mother pig statuses indicating rearing states of the mother pigs whose statuses transition by conducting a breeding event on the mother pigs, and history information indicating timings at which the mother pig statuses have transitioned; and
a production group registration step of assigning, by a production group registration unit, a production group identifier for identifying the mother pigs as a production group on which an identical breeding event is to be conducted at an identical timing in association with the mother pig identifiers extracted by the mother pig extraction unit.

19. A livestock information management program for managing breeding of multiple mother pigs, the program **characterized by** causing a computer to execute:
a mother pig extraction step of extracting, by a mother pig extraction unit, multiple mother pig identifiers in an identical mother pig status by using mother pig identifiers for identifying the respective mother pigs, mother pig statuses indicating rearing states of the mother pigs whose statuses transition by conducting a breeding event on the mother pigs, and history information indicating timings at which the mother pig statuses have transitioned, and
a production group registration step of assigning, by a production group registration unit, a production group identifier for identifying the mother pigs as a production group on which an identical breeding event is to be conducted at an identical timing in association with the mother pig identifiers extracted by the mother pig extraction unit.
